(19) Europäisches Patentamt / European Patent Office / Office européen des brevets

(11) **EP 4 345 658 B1**

(12) **EUROPÄISCHE PATENTSCHRIFT**

(45) Veröffentlichungstag und Bekanntmachung des Hinweises auf die Patenterteilung:
**13.08.2025   Patentblatt 2025/33**

(21) Anmeldenummer: **22199099.7**

(22) Anmeldetag: **30.09.2022**

(51) Internationale Patentklassifikation (IPC):
*G06F 18/25* (2023.01)     *G06V 10/25* (2022.01)
*G06V 10/44* (2022.01)     *G06V 10/82* (2022.01)
*G06V 20/69* (2022.01)

(52) Gemeinsame Patentklassifikation (CPC):
**G06V 10/82; G06F 18/254; G06V 10/25;
G06V 10/44; G06V 20/69; G06V 20/698**

(54) **VERFAHREN UND VORRICHTUNG ZUR DETEKTION EINER PRÄSENZ EINES FLUORESZENZMUSTERS AUF EINEM IMMUNFLUORESZENZBILD EINES BIOLOGISCHEN ZELLSUBSTRATS**

METHOD AND DEVICE FOR DETECTING PRESENCE OF FLUORESCENT PATTERN ON IMMUNOFLUORESCENCE IMAGE OF BIOLOGICAL CELL SUBSTRATE

PROCÉDÉ ET DISPOSITIF DE DÉTECTION D'UNE PRÉSENCE D'UN MOTIF FLUORESCENT SUR UNE IMAGE IMMUNOFLUORESCENTE D'UN SUBSTRAT DE CELLULE BIOLOGIQUE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(43) Veröffentlichungstag der Anmeldung:
**03.04.2024   Patentblatt 2024/14**

(73) Patentinhaber: **EUROIMMUN Medizinische
Labordiagnostika AG
23560 Lübeck (DE)**

(72) Erfinder:
• **KRAUTH, Jens
23560 Lübeck (DE)**
• **HOCKE, Jens
23560 Lübeck (DE)**
• **GERLACH, Stefan
23560 Lübeck (DE)**
• **KRAUSE, Christopher
23560 Lübeck (DE)**
• **HAHN, Melanie
23560 Lübeck (DE)**
• **VOIGT, Jörn
23560 Lübeck (DE)**

(56) Entgegenhaltungen:
**EP-A1- 3 971 827      EP-A1- 4 030 340
CN-A- 108 364 006**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein digitales Bildverarbeitungsverfahren sowie eine Vorrichtung zur Detektion einer Präsenz eines Fluoreszenzmusters auf einem Immunfluoreszenzbild eines biologischen Zellsubstrats. Die Erfindung betrifft ferner ein Computerprogrammprodukt, ein Datenträgersignal sowie eine Datennetzwerkvorrichtung.

Immunfluoreszenzbilder von biologischen Zellsubstraten können durch Bilderfassungseinheiten wie beispielsweise Mikroskope erfasst werden. Zum Zwecke einer Unterstützung einer diagnostischen Fragestellung kann eine Information bezüglich einer Präsenz eines zu erwartenden Fluoreszenzmusters auf einem Fluoreszenzbild vorteilhaft sein.

Eine Immunfluoreszenzmikroskopie bzw. indirekte Immunfluoreszenzmikroskopie ist ein in-vitro-Test für eine Bestimmung einer Präsenz humaner Antikörper gegen bestimmte Antigene, um eine diagnostische Fragestellung beantworten bzw. einschätzen zu können. Derartige Antigene sind beispielsweise in bestimmten Bereichen von biologischen Zellsubstraten. Als Substrat dient also ein Zellsubstrat, welches mit einer Patientenprobe in Form von Blut oder verdünntem Blut oder aber Blutserum oder verdünntem Blutserum inkubiert wird. Die Patientenprobe weist also potenziell bestimmte primäre Antikörper auf, welche Ausdruck eines Vorhandenseins einer Erkrankung des Patienten sein können. Solche primären bzw. spezifischen Antikörper können dann an Antigene des Substrats binden. Derartig gebundene primäre Antikörper können dann dadurch markiert werden, dass in einem weiteren Inkubationsschritt sogenannte sekundäre Antikörper, vorzugsweise Anti-Human-Antikörper, an die gebundenen primären Antikörper anbinden und später dadurch sichtbar gemacht werden können, dass die sekundären Antikörper mit einem Fluoreszenzfarbstoff markiert sind. Ein solcher Fluoreszenzfarbstoff ist vorzugsweise ein grüner Fluoreszenzfarbstoff, insbesondere der Fluoreszenzfarbstoff FITC. Eine solche Bindung eines primären Antikörpers gemeinsam mit einem fluoreszenzmarkierten sekundären Antikörper kann dann später dadurch sichtbar gemacht werden, dass das Substrat mit Anregungslicht einer bestimmten Wellenlänge bestrahlt wird und somit die gebundenen Fluoreszenzfarbstoffe zur Emission von Fluoreszenzstrahlung angeregt werden.

Abhängig von der diagnostischen Fragestellung kann auf eine Präsenz eines oder ganz bestimmter Fluoreszenzmustertypen auf bestimmten Substratbereichen abgestellt werden. Es ergibt sich also die Aufgabe, mittels digitaler Bildverarbeitung im Zuge einer Immunfluoreszenzmikroskopie bei einem in der vorgeschriebenen Weise inkubierten Zellsubstrat eine Präsenz eines potenziellen Fluoreszenzmusters in einem Fluoreszenzbild einer Immunfluoreszenzmikroskopie zu detektieren.

Ein biologisches Zellsubstrat kann hierbei insbesondere ein Zellausstrich von Zelllinien, eine Kolonie von Zelllinien und/oder biologisches Gewebe mit biologischen Gewebezellen, insbesondere aus einem Gewebeschnitt, sein.

Im indirekten Immunfluoreszenztest (IIFT) werden vorzugsweise zum Nachweis von Autoantikörpern gegen Pankreasinseln (islet cell antibodies = ICA) gefrierfixierte Gewebsschnitte des Substrats Primatenpankreas verwendet. Das Pankreas kann in einen exokrinen und in einen endokrinen Bereich unterteilt werden. Antikörper gegen Inselzellen reagieren nur mit dem endokrinen Teil des Organs, den Langerhans'schen Inseln, auch "Islets" genannt, und sind gegen die Insulin-produzierenden Betazellen gerichtet. Als Antigene wurden die 65 kDa-Isoform des Enzyms Glutamat-Decarboxylase (GAD65), die Tyrosinphosphatasehomologen IA-2-Proteine (IA-2$\alpha$ und IA-2$\beta$), der Kationentransporter ZnT8, Insulin und die Insulinvorstufe Proinsulin identifiziert.

Die resultierende Zerstörung der Betazellen führt zum Insulinmangel und so zu Diabetes mellitus Typ 1. Bei 70 bis 80 % der Typ-1-Diabetiker können zum Zeitpunkt der klinischen Manifestation Inselzellantikörper nachgewiesen werden. Sie sind daher ein wichtiges Unterscheidungsmerkmal zwischen Diabetes mellitus Typ 1 und Diabetes mellitus Typ 2 (Altersdiabetes).

Bezüglich einer besonderen Verlaufsform des Diabetes mellitus Typ 1, dem versteckten Autoimmundiabetes im Erwachsenenalter (latent autoimmune diabetes in adults = LADA), dienen Inselzellantikörper auch als Kriterium für die Insulinpflichtigkeit der Patienten. Ein LADA liegt bei 3 bis 12 % der Patienten mit phänotypischem Diabetes mellitus Typ 2 vor, der durch Insulinresistenz und eine gestörte Insulinsekretion der $\beta$-Zellen charakterisiert ist.

Bei 90 % der Patienten sind Inselzellantikörper bereits Jahre vor der Diabetes-Manifestation nachweisbar. Sie gelten als Marker der sogenannten prädiabetischen Phase. So kann eine frühzeitige Identifizierung der Personen mit erhöhtem Erkrankungsrisiko sichergestellt werden. Durch geeignete Interventionen lässt sich damit unter Umständen ein Ausbruch der Erkrankung verhindern.

Der Titer für Inselzellantikörper nimmt mit Fortschreiten der Erkrankung wieder ab. Eine sehr hohe Konzentration von Autoantikörpern gegen Inselzellen (GAD65) kann als Hinweis auf Stiff-Person-Syndrom, Kleinhirndegeneration und limbische Enzephalitis sein.

Im indirekten Immunfluoreszenztest zeigen die Langerhans'schen Inseln des Pankreasgewebes bei positivem Ergebnis eine feinfleckige, glatte bis körnige Fluoreszenz. Es kann im indirekten Immunfluoreszenztest (IIFT) ferner vorzugsweise ein Substrat verwendet werden, bei welchem mehrere *Crithidia luciliae* Zellen auf dem Substrat fixiert sind. Ein solches Substrat ist insbesondere ein Zellausstrich. Eine solche Fixierung kann beispielsweise mittels Ethanol vorgenommen sein. Das Substrat kann dann verwendet werden zum Detektieren einer Bindung von Autoantikörpern einer Patientenprobe an doppelsträngige Desoxyribonukleinsäure (DNS). Der Nachweis von Autoantikörpern gegen Desoxyribonucleinsäuren (DNS) ist beispielsweise für die Diagnose des SLE (Synonym: *Lupus erythematodes disseminatus*) von entscheidender Bedeutung. Hierbei müssen grundsätzlich zwei Typen unterschieden werden: Antikörper gegen dsDNS und Antikörper gegen einzelsträngige, denaturierte DNS (Einzelstrang-DNS, ssDNS). Antikörper gegen dsDNS reagieren mit Epitopen, die im Desoxyribosephosphat-Gerüst der DNS liegen. Dagegen binden sich Antikörper gegen ssDNS vorwiegend an Epitope aus dem Bereich der Purin bzw. Pyrimidinbasen. Sie können aber auch Epitope des Desoxyribosephosphat-Gerüsts erkennen. Anti-dsDNS-Antikörper findet man fast ausschließlich beim SLE. Ihre Prävalenz beträgt, je nach Nachweismethode und Krankheitsaktivität, 20 % bis 90 %. Anti-dsDNS-Antikörper werden mitunter auch bei Patienten mit anderen Autoimmunerkrankungen und Infektionen sowie in seltenen Fällen bei klinisch gesunden Personen nachgewiesen. Letztere entwickeln in 85 % der Fälle einen SLE innerhalb von 5 Jahren nach dem Anti-dsDNS-Erstnachweis. Allerdings lässt sich ein SLE nicht ganz ausschließen, wenn keine Antikörper gegen dsDNS vorliegen. Der SLE ist eine systemische Autoimmunerkrankung aus der Gruppe der Kollagenosen. Die Diagnose richtet sich nach den 1997 modifizierten 11 Kriterien des American College of Rheumatology (ACR). Bei Vorhandensein von 4 der 11 Kriterien kann mit 80- bis 90 %-iger Sicherheit die Diagnose eines SLE gestellt werden. Eine indirekte Immunfluoreszenz kann also ein in-vitro-Test für die Bestimmung humaner Antikörper gegen dsDNS sein. Es können beispielsweise sogenannte BIOCHIPs als Substrate dienen, die mit *Crithidia luciliae* Ausstrichen beschichtet sind. Diese werden beispielsweise mit verdünnten Patientenproben inkubiert. Bei positiven Reaktionen binden sich spezifische Antikörper an die Antigene. Gebundene Antikörper (IgG) werden in einem zweiten Inkubationsschritt beispielsweise mit Fluorescein-markierten Anti-Human-Antikörpern angefärbt und im Fluoreszenzmikroskop sichtbar gemacht.

Das Dokument EP 3971827 A1 offenbart ein Verfahren zum Detektieren einer Bindung von Antikörpern einer Patientenprobe an ein biologisches Substrat aufweisen *Crithidia luciliae* Zellen und Fluoreszenzmikroskopie, wobei für Teilbilder nur erste Teilkonfidenzmaße ermittelt werden.

Das Dokument EP 4030340 A1 offenbart ein Verfahren zum Erkennen von Fluoreszenzmustern in Teilbereichen eines Immunfluoreszenzbildes eines Zellsubstrats mittels eines neuronalen Netzes, welches Teilbilder prozessiert.

Das Dokument CN 108364006 A bezieht sich auf eine Auswertung von medizinischen Bildern bzgl. erkrankten Gewebes anstelle von Fluoreszenzbildern.

Aufgabe der vorliegenden Erfindung ist es, ein automatisiertes Bildverarbeitungsverfahren für eine Analyse eines Immunfluoreszenzbildes eines biologischen Zellsubstrats bereitzustellen, welches eine Präsenz eines Fluoreszenzmusters auf dem Zellsubstrat detektieren kann.

Die erfindungsgemäße Aufgabe wird gelöst durch das erfindungsgemäße Verfahren nach dem Patentanspruch 1.

Erfindungsgemäß weist das Verfahren verschiedene Schritte auf.

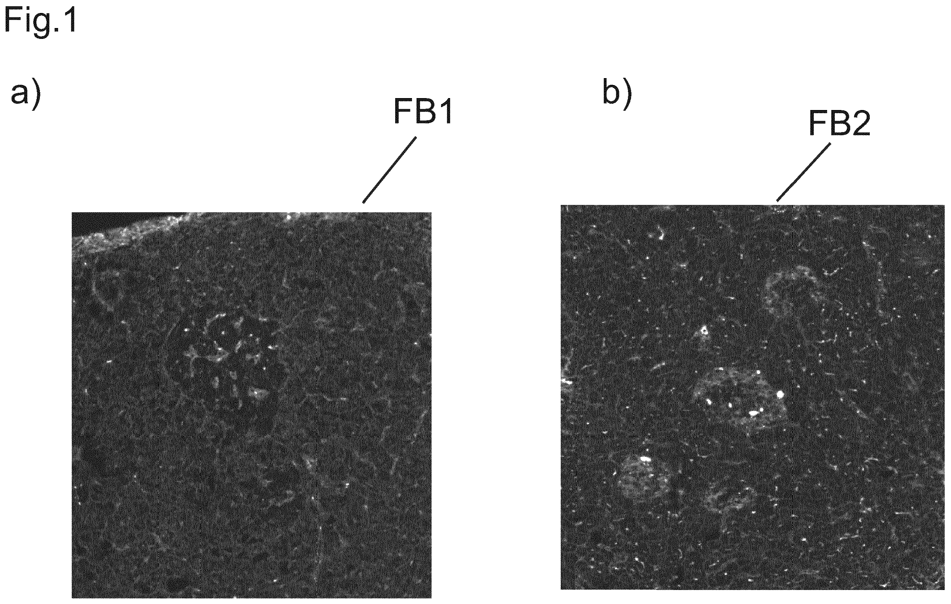

Die Figur 1A zeigt ein beispielhaftes Fluoreszenzbild FB1, in diesem Beispiel eines Gewebeschnittes eines Pankreas. In dem Beispiel des Fluoreszenzbild FB1 ist ein zu erwartendes Fluoreszenzmuster nicht präsent. Die Figur 1B zeigt ein weiteres Fluoreszenzbild FB2, ebenfalls in diesem Fall eines Pankreas, wobei hier ein zu erwartendes Fluoreszenzmuster präsent ist.

Für das Beispiel eines Substrats in Form eines Pankreasschnittes ist auf einen bestimmten bzw. relevanten Teilbereich abzustellen, welches die sogenannten Inseln bzw. Islets sind.

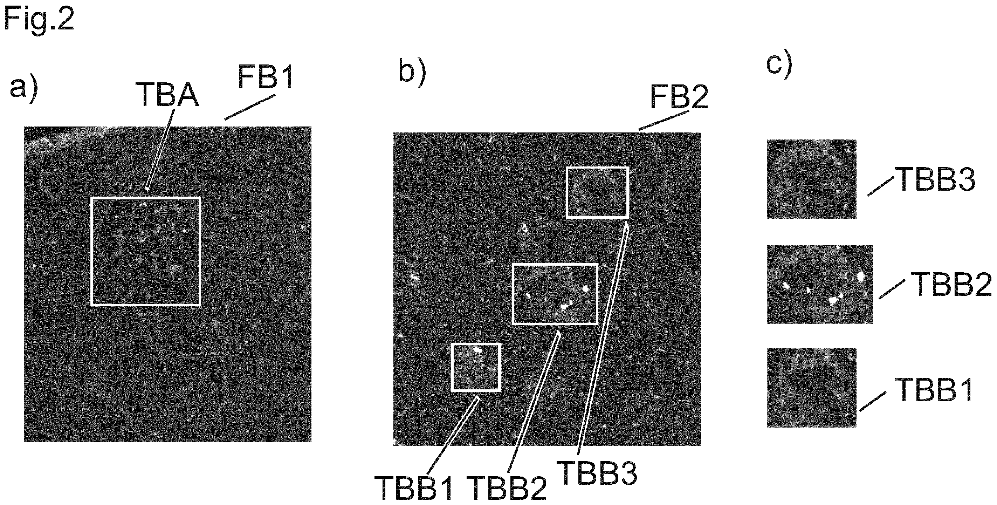

Die Figur 2A zeigt hierfür für das Fluoreszenzbild FB1 einen Bereich TBA als einen Teilbildbereich, welcher ein Islet bzw. eine Insel im Wesentlichen aufweist. In dem Bereich TBA ist kein zu erwartendes Fluoreszenzmuster präsent. Die Figur 2B zeigt hierzu für das Fluoreszenzbild FB2 Teilbildbereiche TBB1, TBB2, TBB3 mit entsprechenden Islets bzw. Inseln, auf welchen ein zu erwartendes Fluoreszenzmuster präsent ist. Diese Teilbildbereiche TBB1, ..., TBB3 sind noch einmal in der Figur 2C separat und vergrößert dargestellt.

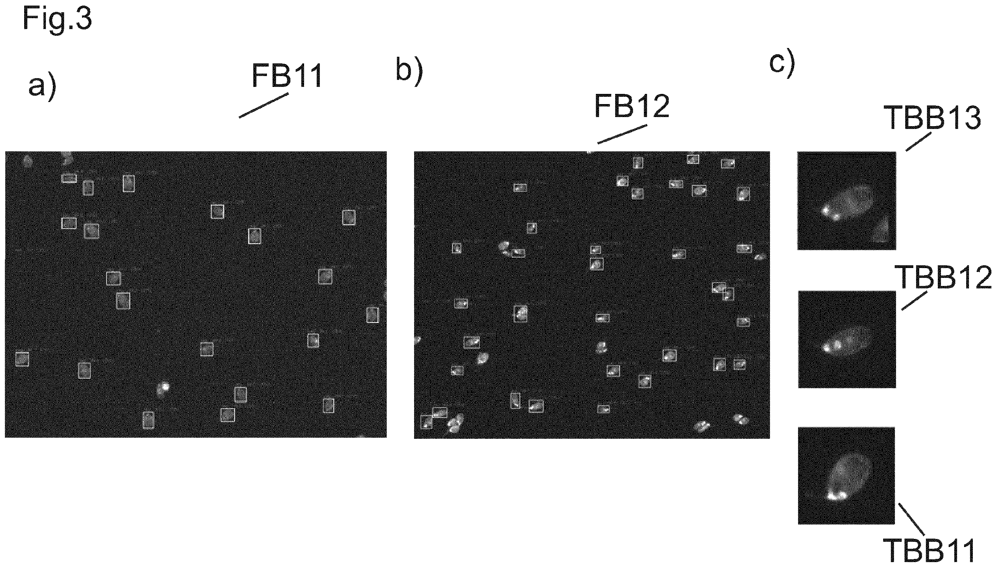

Die Figur 3A zeigt für das Beispiel eines Zellsubstrats in Form eines Zellausstrichs von *Crithidia luciliae* Zellen ein Fluoreszenzbild FB11, bei welchem im Wesentlichen kein zu erwartendes Fluoreszenzmuster präsent ist. Die Figur 3B zeigt ein Beispiel eines Fluoreszenzbildes mit einem Zellsubstrat in Form von einem Zellausstrich mit *Crithidia luciliae* Zellen, bei welchem als das Bild FB12 ein zu erwartendes Fluoreszenzmuster präsent ist. Relevante Teilbereiche bzw. Teilbildbereiche sind hierbei in dem Fluoreszenzbild FB12 durch Rechtecke indiziert. Die relevanten Teilbereiche weisen hierbei insbesondere einen Kinetoplasten einer *Crithidia Luciliae* Zelle auf.

Figur 3C zeigt beispielhafte Teilbildbereiche TBB11, TBB12, TBB13 aus dem Fluoreszenzbild FB12, welche in den relevanten Bereichen in Form der *Crithidia luciliae* Zellen ein zu erwartendes Fluoreszenzmuster präsent ist. Dieses ist insbesondere ein Leuchten des Kinetoplasten.

Erfindungsgemäß erfolgt nach dem Bereitstellen des Fluoreszenzbildes ein Bestimmen jeweiliger Lokalisationsinformationen, welche jeweilige Lokalisationen jeweiliger relevanter Teilbereiche des Zellsubstrats in dem Fluoreszenzbild indizieren. Für das Beispiel des Pankreas sind die relevanten Teilbereiche eben die sogenannten Islets bzw. Inseln, da auf diesen ein zu erwartendes Fluoreszenzmuster detektiert werden muss oder aber festgestellt werden muss, ob das zu erwartende Fluoreszenzmuster nicht präsent ist. Eine solche Lokalisationsinformationen kann beispielsweise in Form eines Rechtecks, wie in der Figur 2B zu sehen, für einen jeweiligen relevanten Teilbereich bestimmt werden.

Ferner erfolgt erfindungsgemäß ein Bestimmen jeweiliger erster Teil-Konfidenzmaße jeweiliger Präsenzen des Fluoreszenzmusters auf den jeweiligen Teilbereichen auf Basis des gesamten Fluoreszenzbildes. Solche Teilbereiche korrespondieren beispielsweise zu den Teilbildbereichen TBB1, TBB2, TBB3 aus der Figur 2B.

Das Bestimmen der Lokalisationsinformationen sowie das Bestimmen der jeweiligen ersten Teil-Konfidenzmaße erfolgt mittels eines ersten neuronalen Netzes auf Basis des gesamten Fluoreszenzbildes. Insbesondere erfolgt dieses Bestimmen der Lokalisationsinformationen und der ersten Teil-Konfidenzmaße gleichzeitig durch das erste neuronale Netz, sodass das erste neuronale Netz insbesondere ein kombiniertes neuronales Netz für beide Aufgaben dieser Bestimmung ist.

Es werden erfindungsgemäß die jeweiligen Teilbildbereiche, welche zu den jeweiligen Teilbereichen des Zellsubstrats korrespondieren, aus dem Fluoreszenzbild extrahiert. Dieses erfolgt auf Basis der zuvor gewonnenen Lokalisationsinformationen. Derartige Teilbildbereiche sind in der Figur 2C als Teilbildbereiche TBB1, TBB2, TBB3 beispielhaft dargestellt.

Ferner erfolgt erfindungsgemäß auf Basis der jeweiligen Teilbildbereiche ein Bestimmen jeweiliger zweiter Teil-Konfidenzmaße bezüglich jeweiliger Präsenzen des Fluoreszenzmusters auf den jeweiligen Teilbereichen mittels eines zweiten neuronalen Netzes. Es werden also beispielsweise die Teilbildbereiche TBB1, TBB2, TBB3 aus der Figur 2C mittels eines zweiten neuronalen Netzes analysiert. Insbesondere erfolgt das Bestimmen der jeweiligen zweiten Teil-Konfidenzmaße mittels des zweiten neuronalen Netzes in der Weise, dass jeder der jeweiligen Teilbildbereiche für sich separat durch das zweite neuronale Netz analysiert bzw. prozessiert wird, um ein jeweiliges zweites Teil-Konfidenzmaß zu bestimmen.

Erfindungsgemäß erfolgt schließlich ein Bestimmen eines Konfidenzmaßes der Präsenz des Fluoreszenzmusters in dem Fluoreszenzbild auf Basis der ersten Teil-Konfidenzmaße und der zweiten Teil-Konfidenzmaße.

Mögliche Vorteile der Erfindung werden nun im Weiteren genauer erläutert.

Die prinzipielle Aufgabe, eine Präsenz eines Fluoreszenzmusters auf einem großen, gesamten Fluoreszenzbild zu detektieren, könnte prinzipiell durch ein einziges neuronales Netz geleistet werden, indem solche großen Gesamtbilder mit entsprechender Ground Truth Information "positiv" oder "negativ" bzgl. der Präsenz des Fluoreszenzmusters in einer Trainingsphase durch ein solches einzelnes neuronales Netz analysiert werden, um dann eine solche Klassenzugehörigkeit zu den Klassen "positiv" oder "negativ" zu bestimmen. Bei einem solchen Vorgehen müsste dieses einzelne neuronale Netz eine große Menge an Bildinformationen aus einem gesamten Fluoreszenzbild einfließen lassen und berücksichtigen. Dieses würde eine große Komplexität des Netzwerkes erfordern und somit auch eine große Menge an Rechenoperationen, sodass der entsprechende Rechenaufwand eine Herausforderung darstellen kann.

Ferner muss beachtet werden, dass neuronale Netze zur Analyse von großen Gesamtbildern solche Bildmerkmale extrahieren und möglicherweise als relevant einstufen können, welche zwar im Gesamtbild präsent sind, jedoch für die eigentliche Klassifikationsaufgabe des neuronalen Netzes irrelevant sein können. Um die Aufmerksamkeit und die zu erlernenden Bildmerkmale des neuronalen Netzes gezielt auf die nur tatsächlich relevanten Bildstrukturen zu lenken, bietet sich das hier vorgeschlagene Verfahren an, um zunächst in einer ersten Stufe mit einem ersten neuronalen Netz direkt die relevanten Strukturen des gesamten Fluoreszenzbildes hinsichtlich ihrer Lokalisation jeweils zu identifizieren und auch jeweils zu klassifizieren und um dann in einer zweiten Stufe die entsprechenden Teilbildbereiche der relevanten Strukturen jeweils für sich durch ein zweites neuronales Netz zusätzlich zu klassifizieren. Hierbei ergibt sich durch Ermittlung der Klassifikationsergebnisse bzw. der ersten Teil-Konfidenzmaße aus der ersten Stufe in Kombination mit der Ermittlung der Klassifikationsergebnisse bzw. der zweiten Teil-Konfidenzmaße aus der zweiten Stufe und Zusammenführung dieser Ergebnisse in dem finalen Konfidenzmaß der Präsenz des Fluoreszenzmusters in dem gesamten Fluoreszenzbild eine hohe Validität des finalen Konfidenzmaßes. Ein weiterer Vorteil ergibt sich dadurch, dass mittels der Teil-Konfidenzmaße der Teilbereiche bzw. der Teilbildbereiche es möglich ist, die Klassifikationsergebnisse aus den beiden Stufen bzw. den beiden neuronalen Netzen kontrolliert in das finale Konfidenzmaß eingehen zu lassen. Hierdurch ergibt sich bezüglich der Funktion der beiden neuronalen Netze in dem Verfahren eine gute Model Interpretability.

Durch die Ermittlung der relevanten Teilbereiche und ihrer korrespondierenden Teilbildbereiche als einzelne Elemente ergibt sich automatisch die Anzahl der in dem Verfahren verfügbaren Teilergebnisse z.B. in Form von Teil-Konfidenzmaßen, wobei bei einem Gesamtbildansatz eine solche Information bzw. Anzahlinformation nicht vorhanden ist, da es dann nur eine Gesamtausgabe eines einzelnen Konfidenzmaßes bezogen auf das Gesamtbild gibt, vorzugsweise als Aussage "positiv" oder "negativ".

Mit anderen Worten: Das erfindungsgemäße Verfahren weicht explizit von einem Ansatz zur Analyse eines gesamten Fluoreszenzbildes mittels nur eines neuronalen Netzes ab, indem zunächst das erste neuronale Netz Lokalisationsinformationen bestimmt, welche relevante Teilbereiche des Zellsubstrats indizieren. Das zweite neuronale Netz muss dann jeweils nur für sich separat einen solchen Teilbildbereich prozessieren, um in dem jeweiligen Teilbildbereich eine mögliche Präsenz des Fluoreszenzmusters zu detektieren und ein entsprechendes Teil-Konfidenzmaß zu ermitteln.

Das zweite neuronale Netz kann also in seiner Komplexität deutlich reduziert sein gegenüber einer Lösung, in welcher ein einzelnes neuronales Netz das Konfidenzmaß der Präsenz des Fluoreszenzmusters für das gesamte Fluoreszenzbild ermitteln muss.

Das zweite neuronale Netz kann somit auf deutlich kleinere Teilbildbereiche hin trainiert werden und muss lediglich solche Arten von Teilbildern analysieren, welche tatsächlich relevante Teilbereiche eines Zellsubstrats repräsentieren.

Daher kann das zweite neuronalen Netz somit deutlich validere Teil-Konfidenzmaße bezüglich Präsenzen des Fluoreszenzmusters auf den jeweiligen Teilbildern bzw. Teilbereichen ermitteln. Daher kann das zweite neuronalen Netz somit auch ein valideres Konfidenzmaß bezüglich einer Präsenz des Fluoreszenzmusters in dem Fluoreszenzbild bestimmen.

Das erfindungsgemäße Verfahren ist ferner vorteilhaft, da das erste neuronale Netz sowohl die relevanten Teilbereiche mittels der Lokalisationsinformationen bestimmt und eben auch die ersten Teil-Konfidenzmaße bestimmt, insbesondere gleichzeitig. Das erste neuronale Netz ist also insbesondere eines, welches zum gleichzeitigen Bestimmen der ersten Teil-Konfidenzmaße und der Bestimmung der Lokalisationsinformationen trainiert wurde. Solche Netzwerke können hinsichtlich Rechenkomplexität und benötigtem Rechenspeicher besonders effizient sein gegenüber anderen Netzwerken. Solche Netzwerke sind insbesondere als sogenannte "One-Shot Detektoren" implementierbar.

Ferner ist das erfindungsgemäße Verfahren vorteilhaft, da das Konfidenzmaß unter Berücksichtigung bzw. auf Basis der ersten Teil-Konfidenzmaße und der zweiten Teil-Konfidenzmaße bestimmt wird, sodass derartige Informationen bezüglich der Teil-Konfidenzmaße aus beiden neuronalen Netzen einfließen können, um ein besonders verlässliches Konfidenzmaß der Präsenz des Fluoreszenzmusters in dem Fluoreszenzbild zu bestimmen.

Vorzugsweise erfolgt ein Ausgeben des Konfidenzmaßes.

Vorzugsweise erfolgt ein Bestimmen gewichteter Teil-Konfidenzmaße mittels Gewichtung der ersten Teil-Konfidenzmaße und der zweiten Teil-Konfidenzmaße, sowie ein Bestimmen des Konfidenzmaßes auf Basis der gewichteten Teil-Konfidenzmaße.

Vorzugsweise erfolgt ein Bestimmen des Konfidenzmaßes mittels Anwendung eines Schwellenwertes auf die gewichteten Teil-Konfidenzmaße.

Vorzugsweise weist das Verfahren ferner die Schritte auf: Bestimmen jeweiliger Präsenz-Konfidenzmaße, welche zu den jeweiligen Lokalisationen der jeweiligen relevanten Teilbereiche indizieren, zu welchem Grade an den jeweiligen Lokalisationen tatsächlich relevante Teilbereiche des Zellsubstrats präsent sind, mittels des ersten neuronalen Netzes auf Basis des gesamten Fluoreszenzbildes, Feststellen, ob die Lokalisationsinformationen eine Menge mehrerer, sich überlappender Teilbildbereiche indizieren, Behalten der Lokalisationsinformation eines bestimmten Teilbildbereiches aus der Menge der sich überlappenden Teilbildbereiche auf Basis der Präsenz-Konfidenzmaße der sich überlappenden Teilbildbereiche und Verwerfen der Lokalisationsinformationen der anderen Teilbildbereiche aus der Menge der sich überlappenden Teilbildbereiche, sowie Extrahieren jeweiliger Teilbildbereiche aus dem Fluoreszenzbild auf Basis der verbleibenden Lokalisationsinformationen.

Ferner weist das Verfahren insbesondere die Schritte auf: Bestimmen einer Menge von Teilbildbereichen, welche eine Präsenz des Fluoreszenzmusters aufweisen, sowie Ermitteln eines Helligkeitswertes der Präsenz der Fluoreszenzmusters auf dem Immunfluoreszenzbild auf Basis der Menge der Menge von Teilbildbereichen, welche eine Präsenz des Fluoreszenzmusters aufweisen.

Vorgeschlagen wird ferner ein Computerprogrammprodukt, umfassend Befehle, die bei der Ausführung des Programms durch einen Computer diesen veranlassen, das Verfahren zur digitalen Bildverarbeitung durchzuführen.

Vorgeschlagen wird ferner ein Datenträgersignal, welches das Computerprogrammprodukt überträgt.

Vorgeschlagen wird ferner eine Vorrichtung zur Detektion eines Fluoreszenzmusters auf einem Immunfluoreszenzbild eines biologischen Zellsubstrats, aufweisend eine Haltevorrichtung für einen Objektträger mit dem Zellsubstrat, welches mit einer Patientenprobe, aufweisend die Autoantikörper, sowie ferner mit sekundären Antikörpern, welche jeweils mit einem Fluoreszenzstoff markiert sind, inkubiert wurde. Die Vorrichtung weist ferner wenigstens eine Bilderfassungseinheit zum Erfassen eines Fluoreszenzbildes des Zellsubstrats auf. Ferner weist die Vorrichtung wenigstens eine Recheneinheit auf, welche ausgebildet ist, die Schritte auszuführen: Bestimmen jeweiliger Lokalisationsinformationen, welche jeweilige Lokalisationen jeweiliger relevanter Teilbereiche des Zellsubstrats in dem Fluoreszenzbild indizieren, und Bestimmen jeweiliger erster Teil-Konfidenzmaße jeweiliger Präsenzen des Fluoreszenzmusters auf den jeweiligen Teilbereichen mittels eines ersten neuronalen Netzes auf Basis des gesamten Fluoreszenzbildes, Extrahieren jeweiliger Teilbildbereiche, welche zu den jeweiligen Teilbereichen des Zellsubstrats korrespondieren, auf Basis der Lokalisationsinformationen, Bestimmen jeweiliger zweiter Teil-Konfidenzmaße jeweiliger Präsenzen des Fluoreszenzmusters auf den jeweiligen Teilbereichen mittels eines zweiten neuronalen Netzes auf Basis der jeweiligen Teilbildbereiche sowie Bestimmen eines Konfidenzmaßes der Präsenz des Fluoreszenzmusters in dem Fluoreszenzbild auf Basis der ersten Teil-Konfidenzmaße und der zweiten Teil-Konfidenzmaße.

Offenbart wird ferner eine Recheneinheit, welche ausgebildet ist, im Zuge einer digitalen Bildverarbeitung die Schritte auszuführen: Entgegennehmen eines Immunfluoreszenzbildes, welches eine Färbung eines biologischen Zellsubstrats durch einen Fluoreszenzfarbstoff repräsentiert, Bestimmen jeweiliger Lokalisationsinformationen, welche jeweilige Lokalisationen jeweiliger relevanter Teilbereiche des Zellsubstrats in dem Fluoreszenzbild indizieren, und Bestimmen jeweiliger erster Teil-Konfidenzmaße jeweiliger Präsenzen des Fluoreszenzmusters auf den jeweiligen Teilbereichen mittels eines ersten neuronalen Netzes auf Basis des gesamten Fluoreszenzbildes, Extrahieren jeweiliger Teilbildbereiche, welche zu den jeweiligen Teilbereichen des Zellsubstrats korrespondieren, auf Basis der Lokalisationsinformationen, Bestimmen jeweiliger zweiter Teil-Konfidenzmaße jeweiliger Präsenzen des Fluoreszenzmusters auf den jeweiligen Teilbereichen mittels eines zweiten neuronalen Netzes auf Basis der jeweiligen Teilbildbereiche sowie Bestimmen eines Konfidenzmaßes der Präsenz des Fluoreszenzmusters in dem Fluoreszenzbild auf Basis der ersten Teil-Konfidenzmaße und der zweiten Teil-Konfidenzmaße.

Vorgeschlagen wird ferner eine Datennetzwerkvorrichtung, aufweisend wenigstens eine Datenschnittstelle zum Entgegennehmen eines Fluoreszenzbildes, welches eine Färbung eines Zellsubstrats durch einen Fluoreszenzfarbstoff repräsentiert. Die Datennetzvorrichtung weist ferner wenigstens eine Recheneinheit auf, welche ausgebildet ist im Zuge einer digitalen Bildverarbeitung Schritte auszuführen: Bestimmen jeweiliger Lokalisationsinformationen, welche jeweilige Lokalisationen jeweiliger relevanter Teilbereiche des Zellsubstrats in dem Fluoreszenzbild indizieren, und Bestimmen jeweiliger erster Teil-Konfidenzmaße jeweiliger Präsenzen des Fluoreszenzmusters auf den jeweiligen Teilbereichen mittels eines ersten neuronalen Netzes auf Basis des gesamten Fluoreszenzbildes, Extrahieren jeweiliger Teilbildbereiche, welche zu den jeweiligen Teilbereichen des Zellsubstrats korrespondieren, auf Basis der Lokalisationsinformationen, Bestimmen jeweiliger zweiter Teil-Konfidenzmaße jeweiliger Präsenzen des Fluoreszenzmusters auf den jeweiligen Teilbereichen mittels eines zweiten neuronalen Netzes auf Basis der jeweiligen Teilbildbereiche sowie Bestimmen eines Konfidenzmaßes der Präsenz des Fluoreszenzmusters in dem Fluoreszenzbild auf Basis der ersten Teil-Konfidenzmaße und der zweiten Teil-Konfidenzmaße.

Im Folgenden wird die Erfindung anhand spezieller Ausführungsformen ohne Beschränkung des allgemeinen Erfindungsgedankens anhand der Figuren näher erläutert. Dabei zeigen:

Figuren 1 und 2    Immunfluoreszenzbilder sowie Teilbilder eines ersten biologischen Zellsubstrats,

Figur 3    Immunfluoreszenzbilder sowie Teilbilder eines zweiten biologischen Zellsubstrats,

Figur 4    Schritte einer bevorzugten Ausführungsform des erfindungsgemäßen Verfahrens,

Figur 5    bevorzugte Schritte zur Bestimmung des Konfidenzmaßes bei Gewichtung von Teil Konfidenzmaßen,

Figur 6    Schritte des erfindungsgemäßen Verfahrens bezüglich einer weiteren bevorzugten Ausführungsform,

Figur 7    Schritte zur Bestimmung eines Helligkeitsmaßes der Präsenz des Fluoreszenzmusters,

Figur 8    eine bevorzugte Ausgestaltungsform eines zweiten neuronalen Netzes

Figur 9    eine bevorzugte Ausführungsform einer erfindungsgemäßen Vorrichtung,

Figur 10    eine bevorzugte Ausführungsform einer offenbarten Recheneinheit,

Figur 11    eine bevorzugte Ausführungsform einer erfindungsgemäßen Datennetzwerkvorrichtung,

Figur 12    eine Illustration eines Computerprogrammproduktes sowie eines Datenträgersignals,

Figur 13    eine Ergebnistafel von Experimentalergebnissen.

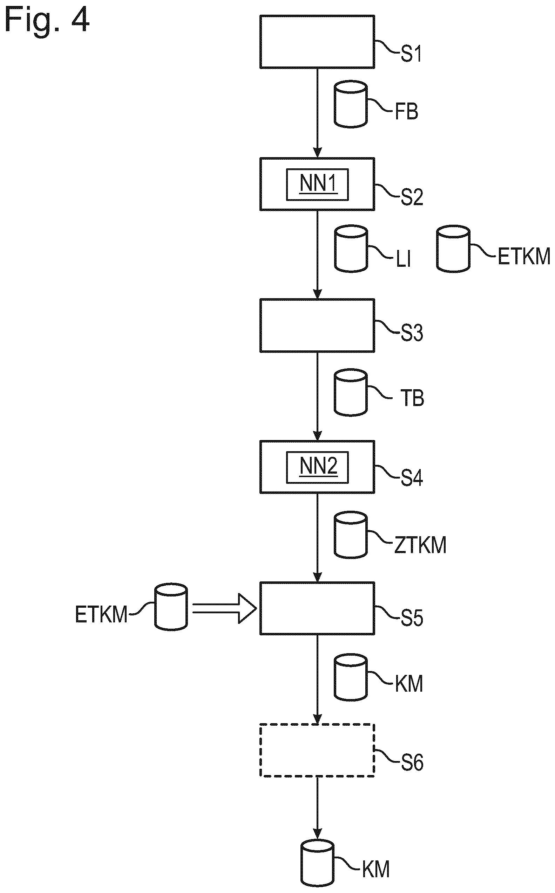

Die Figur 4 zeigt Schritte einer bevorzugten Ausführungsform des erfindungsgemäßen Verfahrens. In einem Schritt S1 erfolgt ein Inkubieren des Zellsubstrats mit einer flüssigen Patientenprobe, welche potenziell primäre Antikörper aufweist, sowie ferner mit sekundären Antikörpern, welche mit einem Fluoreszenzfarbstoff markiert sind. Ferner erfolgt in dem Schritt S1 ein Bestrahlen des Zellsubstrats mit einer Anregungsstrahlung sowie Erfassung des Immunfluoreszenzbildes.

Das Immunfluoreszenzbild kann als ein Datenelement FB durch den Schritt S1 bereitgestellt werden.

Ein solches Immunfluoreszenzbild ist beispielhaft als ein Bild FB1 in der Figur 1A dargestellt. In diesem Fall ist in den relevanten Zellbereichen, hier die Islets bzw. Inseln, des Zellsubstrates, hier in Form eines Pankreasschnittes, nicht präsent. In dem Immunfluoreszenzbild FB2 der Figur 1B ist eine solche Immunfluoreszenz in den relevanten Teilbereichen präsent.

Die Figur 2A zeigt das Immunfluoreszenz Bild FB1 noch einmal mit einem indizierten Teilbereich bzw. einem Teilbildbereich TBA, innerhalb dessen ein relevanter Teilbereich des Zellsubstrates liegt. Im Vergleich zu Teilbereichen bzw. Teilbildbereichen TBB1, TBB2, TBB3 des Fluoreszenzbildes FB2 aus der Figur 2B ist ersichtlich, dass in dem Immunfluoreszenzbild FB1 eine Präsenz eines zu erwartenden Fluoreszenzmusters nicht gegeben ist, jedoch diese Präsenz des Fluoreszenzmusters in den Teilbildbereichen des Immunfluoreszenzbildes FB2 gegeben ist. Die Fluoreszenzmuster der jeweiligen Teilbildbereiche TBB1, TBB2, TBB3 sind noch einmal in vergrößerter Darstellung in Figur 2C zu sehen.

Im Sinne dieser Anmeldung kann ein Teilbild auch als ein Teilbildbereich bezeichnet werden.

Gemäß der Figur 4 erfolgt in dem Schritt S2 das Bestimmen jeweiliger Lokalisationsinformationen, welche jeweilige Lokalisationen jeweiliger relevanter Teilbereiche des Zellsubstrats in dem Fluoreszenzbild indizieren. Dieses erfolgt unter Verwendung eines neuronalen Netzes NN1, welches als ein erstes neuronales Netz bezeichnet werden kann.

Ferner erfolgt in dem Schritt S2 ein insbesondere gleichzeitiges Bestimmen jeweiliger erster Teil-Konfidenzmaße jeweiliger Präsenzen des Fluoreszenzmusters auf den jeweiligen Teilbereichen bzw. jeweiligen relevanten Teilbereichen mittels des neuronalen Netzes NN1 auf Basis des gesamten Fluoreszenzbildes. Dies erfolgt also in insbesondere einer kombinierten bzw. gleichzeitigen Prozessierung.

Die ermittelten Lokalisationsinformationen können als ein kombiniertes Datenelement LI bereitgestellt werden. Die ersten Teil-Konfidenzmaße können als ein Datenelement ETKM bereitgestellt werden.

Eine Lokalisationsinformation indiziert vorzugsweise eine Position eines Teilbereiches in einem Fluoreszenzbild, ferner eine Breiteninformation und ferner eine Höheninformation des Teilbereiches. Vorzugsweise kann die Lokalisationsinformation also ein Rechteck an einer bestimmten Position des Fluoreszenzbildes sowie die Höhe und die Breite des Rechtecks indizieren.

Eine solche Lokalisationsinformation für einen Teilbereich bzw. einen Teilbildbereich kann als ein Vektor n gegeben sein,

$$\vec{n} = \begin{bmatrix} n1 \\ n2 \\ n3 \\ n4 \end{bmatrix}$$

wobei der Eintrag n1 eine X-Position und der Eintrag n2 eine Y-Position eines Rechtecks in dem Fluoreszenzbild indizieren kann sowie ferner der Eintrag n3 eine Breite des Rechtecks und der Eintrag n4 eine Höhe des Rechtecks.

Ein erstes Teil-Konfidenzmaß ETKM für einen Teilbereich, beispielsweise einen Teilbereich TBB1 aus der Figur 2C, kann beispielsweise durch einen Vektor o

$$\vec{o} = \begin{bmatrix} o1 \\ o2 \end{bmatrix}$$

gegeben sein, wobei der Eintrag o1 eine Wahrscheinlichkeit angibt, dass der Teilbildbereich zu einer Klasse einer positiven Fluoreszenz des Musters gehört und wobei der Eintrag o2 eine Wahrscheinlichkeit indiziert, dass Teilbereich zu einer Klasse einer negativen Fluoreszenz des Musters bzw. einer Abwesenheit des Fluoreszenzmusters gehört.

Die Lokalisationsinformationen können also je Teilbildbereich ein jeweiliger Vektor n sein und die ersten Teil-Konfidenzmaße können also je Teilbildbereich ein jeweiliger Vektor o sein.

Das erste neuronale Netz ist vorzugsweise ein sogenanntes Object Detection Network. Besonders bevorzugt ist das erste neuronale Netz, ein Object Detection Network des Typs eines sogenannten One-Shot Detectors. Insbesondere ist das erste neuronale Netz ein sogenanntes YOLO Network.

Ein solches Netzwerk kann beispielsweise dadurch trainiert werden, dass einer Eingangsebene bzw. einem Input-Layer des neuronalen Netzes gesamte Fluoreszenzbilder präsentiert werden, wobei ferner als Ground Truth Information Lokalisationsinformationen jeweiliger Teilbereiche des Zellsubstrats bereitgestellt werden als auch Ground Truth Informationen bezüglich einer Klassenzugehörigkeit eines korrespondierenden Teilbildbereiches des Teilbereiches für die Klassen "positive Fluoreszenz" oder "negative Fluoreszenz".

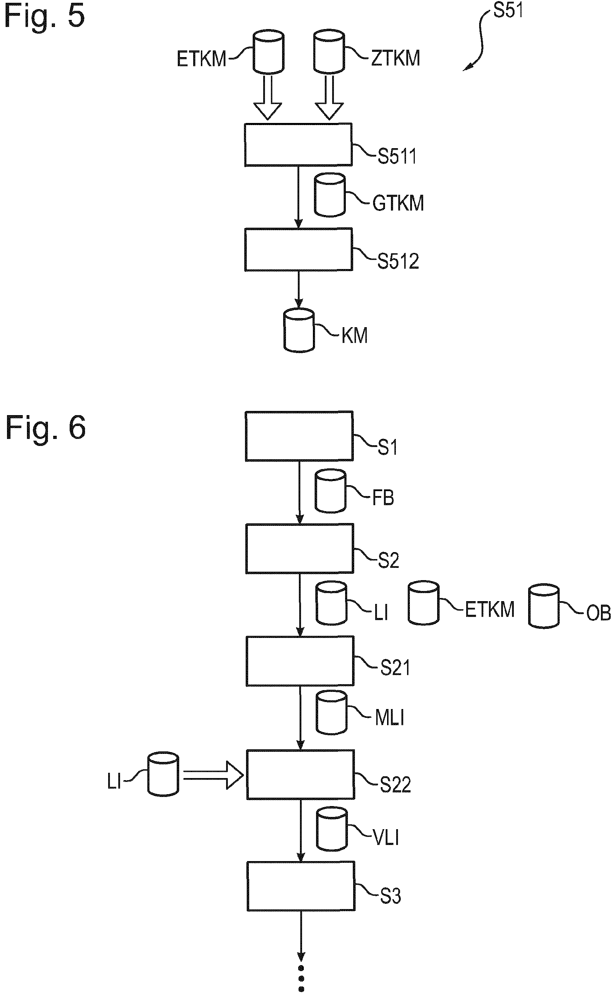

Vorzugsweise ermittelt das erste neuronale Netz NN1 auf Basis des gesamten Fluoreszenzbildes ferner eine weitere Information OB, auch gezeigt in Figur 6. Dies ist eine Information OB, welche je Lokalisation bzw. je Lokalisationsinformation ein Präsenz-Konfidenzmaß ist, welches zu einer jeweiligen Lokalisation eines jeweiligen relevanten Teilbereiches indiziert, zu welchem Grade an der jeweiligen Lokalisation tatsächlich ein relevanter Teilbereich des Zellsubstrats präsent ist. Diese Information OB kann für einen jeweiligen Teilbildbereich mittels eines jeweiligen Skalarwertes c ausgegeben werden.

Das erste neuronale Netz gibt also vorzugsweise je detektiertem relevanten Teilbildbereich mit Index k=1... K ein Tupel $\{c, \vec{o}, \vec{n}\}$ aus. Das Training des neuronalen Netzes kann also insbesondere unter Bereitstellung ganzer Fluoreszenzbilder als auch Ground Truth Informationen in Form entsprechender Tupel $\{c, \vec{o}, \vec{n}\}$ je Teilbereich erfolgen.

Detaillierte Strukturen eines solchen ersten neuronalen Netzes für die besondere Ausführungsform eines Yolo-Netzwerkes sind zu finden in J. Redmon, S. Divvala, R. Girshick and A. Farhadi, "You Only Look Once: Unified, Real-Time Object Detection," 2016 IEEE Conference on Computer Vision and Pattern Recognition (CVPR), 2016, pp. 779-788, doi: 10.1109/CVPR.2016.91.

Das Konfidenzmaß c ist dort als so genannter "confidence" bezeichnet. Der Vektor n ist dort gegeben durch Werte Bildposition x, y als auch Breite b und Höhe h. Die Klassenwahrscheinlichkeiten o1 bzw. o2 sind dort gegeben als sogenannte Conditional Class Probabilities "C".

In einem Schritt S3 erfolgt ein Extrahieren jeweiliger Teilbildbereiche, welche zu den jeweiligen Teilbereichen des Zellsubstrats korrespondieren, aus dem Fluoreszenzbild auf Basis der Lokalisationsinformationen. Diese sind beispielsweise die Teilbildbereiche aus der Figur 2C. Diese Teilbildbereiche können als kombiniertes Datenelement TB bereitgestellt werden. Vorzugsweise ist ein extrahierter Teilbildbereich identisch zu dem entsprechenden Teilbereich, welcher durch eine entsprechende Lokalisationsinformation indiziert wird. Insbesondere kann ein Teilbildbereich eine gewisse Abweichung von dem Teilbereich aufweisen, insbesondere im Randbereich.

In einem Schritt S4 erfolgt ferner ein Bestimmen der jeweiligen zweiten Teil-Konfidenzmaße jeweiliger Präsenzen des Fluoreszenzmusters auf den jeweiligen Teilbereichen mittels eines zweiten neuronalen Netzes NN2 auf Basis der jeweiligen korrespondierenden Teilbildbereiche.

Der Schritt S4 kann diese zweiten Teil-Konfidenzmaße dann als ein Datenelement ZTKM bereitstellen.

Das zweite neuronale Netz gibt je Teilbildbereich vorzugsweise einen Vektor d mit einer Klassenwahrscheinlichkeit für die jeweiligen Klassen "Fluoreszenz positiv" oder "Fluoreszenz negativ" aus. Insbesondere kann also der Vektor d ein Vektor

$$\vec{d} = \begin{bmatrix} d1 \\ d2 \end{bmatrix}$$

sein, wobei der Eintrag d1 eine Klassenwahrscheinlichkeit "Fluoreszenz positiv" und der Eintrag d2 eine Klassenwahrscheinlichkeit "Fluoreszenz negativ" sein kann. Das zweite Teil-Konfidenzmaß kann also für einen Teilbildbereich ein solcher Vektor d sein.

In dem Schritt S5 wird dann auf Basis der zweiten Teil-Konfidenzmaße ZTKM als auch der ersten Teil-Konfidenzmaße ETKM, welche in den Schritt S5 eingehen, das Konfidenzmaß KM der Präsenz des Fluoreszenzmusters bestimmt. Vorzugsweise wird in einem Schritt S6 das Konfidenzmaß KM ausgegeben. Eine solche Ausgabe kann vorzugsweise in Form einer Ausgabe eines Datenelementes über eine Datenschnittstelle erfolgen oder besonders bevorzugt mittels einer optischen Ausgabe auf einer Anzeige einer Recheneinheit wie beispielsweise einem Computermonitor.

Vorzugsweise kann die Bestimmung des Konfidenzmaßes KM dadurch erfolgen, dass für ein jeweiliges Teilbild bzw. einen jeweiligen Teilbildbereich mit Index k=1... K eine jeweilige Mittelung des jeweiligen ersten Teil-Konfidenzmaßes und des jeweiligen zweiten Teil-Konfidenzmaßes erfolgt, sodass sich jeweilige gemittelte Teil-Konfidenzmaße für jeweilige Teilbildbereiche ergeben. Es können dann diese gemittelten Teil-Konfidenzmaße noch einmal über alle Teilbildbereiche bzw. alle Indizes k=1...K gemittelt werden, um das Konfidenzmaß der Präsenz des Fluoreszenzmusters zu bestimmen.

Gemäß der Figur 5 kann alternativ zu dem Schritt S5 ein modifizierter Schritt S51 mit Teilschritten S511 und S512 durchgeführt werden. In dem Schritt S511 gehen die ersten Teil-Konfidenzmaße ETKM und die zweiten Teil-Konfidenzmaße ZTKM ein. In dem ersten Schritt erfolgt ein Bestimmen gewichteter Teil-Konfidenzmaße mittels Gewichtung der ersten Teil-Konfidenzmaße ETKM und der zweiten Teil-Konfidenzmaße ZTKM. Die gewichteten Teil-Konfidenzmaße können als ein Datenelement GTKM bereitgestellt werden. Das Bestimmen des Konfidenzmaßes KM erfolgt dann in dem Schritt S512 auf Basis der gewichteten Teil-Konfidenzmaße GTKM.

Gemäß einer bevorzugten Ausführungsform erfolgt also eine Gewichtung je Teilbildbereich des ersten Teil-Konfidenzmaßes und des zweiten Teil-Konfidenzmaßes. Dies kann also als eine Bestimmung jeweiliger gewichteter Teil-Konfidenzmaße mittels Gewichtung der jeweiligen ersten Teil-Konfidenzmaße und der jeweiligen zweiten Teil-Konfidenzmaße bezeichnet werden.

In einer besonderen Ausführungsform erfolgt also eine Gewichtung mit Gewichtungsfaktoren w1 und w2 je Teilbildbereich gemäß

$$\vec{r} = w1 * \vec{o} + w2 * \vec{d} = \begin{bmatrix} r1 \\ r2 \end{bmatrix}$$

zur Ermittlung eines Vektors

$$\vec{r_k}$$

je Teilbildbereich k=1... K.

Diese gewichteten Teil-Konfidenzmaße in Form der Vektoren $\vec{r_k}$ je Teilbildbereich mit Index k=1...K können dann herangezogen werden, um das Konfidenzmaß KM zu bestimmen. Insbesondere können vorzugsweise die Werte r1 je Teilbildbereich betrachtet werden, welche eine Klassenwahrscheinlichkeit für die Klasse "Fluoreszenz positiv" repräsentieren. Diese Werte R1 können dann jeweils einer Anwendung eines Schwellenwertes unterzogen werden. Es erfolgt also das Bestimmen des Konfidenzmaßes mit der Anwendung eines Schwellenwertes auf die gewichteten Teil-Konfidenzmaße.

Vorzugsweise wird ein Teilbildbereich dann als zu der Klasse "Fluoreszenz positiv" zugeordnet, wenn der entsprechende Wert r1 größer als der Schwellenwert ist. Beispielsweise kann der Wert R1 im Wertebereich von [0...1] liegen und ein Schwellenwert von 0,6 angewendet werden.

Teilbildbereiche, für welche der Wert r1 nicht den Schwellenwert überschreitet, werden dann als zugehörig zu der Klasse "Fluoreszenz negativ" klassifiziert.

Es kann dann vorzugsweise ein sogenanntes Majority Voting erfolgen. Bei diesem Majority Voting kann dann das Konfidenzmaß insbesondere als eine Aussage "ja"/"nein" ermittelt werden, wobei die Mehrheit der Teilbildbereiche entscheidend ist. Gibt es also mehr Teilbildbereiche, für welche das Fluoreszenzmuster als präsent erkannt wurde bzw. für welche die Klasse "Fluoreszenz positiv" erkannt wurde, so ist das Konfidenzmaß "ja". Umgekehrtes gilt für die Möglichkeit des Konfidenzmaßes "nein".

Der Vorteil des Verfahrens einer Anwendung eines Schwellenwertes auf die gewichteten Teil-Konfidenzmaße ist, dass in dem Verfahren eingestellt werden kann, ab welchem Konfidenzmaßwert das gesamte Fluoreszenzbild an sich als positiv bewertet wird bzw. das erwartete Fluoreszenzmuster als präsent bewertet wird. Dies ist insbesondere abhängig von den einzelnen gewichteten Teil-Konfidenzmaßen der Teilbildbereiche einstellbar.

Aufgrund einer sich ergebenden Grid-Struktur im Zusammenhang mit einem sogenannten Striding eines neuronalen Netzes wie dem z.b. neuronalen Netz NN1 ist es möglich, dass ein solches erstes neuronales Netz NN1 mehrere Lokalisationsinformationen bestimmt, welche jeweilige Teilbereiche indizieren, welche sich überlappen. Eine räumliche Trennung von Teilbereichen bzw. Teilbildbereichen, wie bei den Teilbildbereichen TBB1, TBB2, TBB3 aus Figur 2C, muss nicht unbedingt gegeben sein. Es kann auch vorkommen, dass Teilbildbereiche bestimmt werden, welche sich in ihrer Fläche zumindest teilweise überlappen. Insbesondere kann sich eine Überlappung von mehreren Teilbereichen ergeben, welche einen gleichen relevanten Teilbereich, z.B. ein gleiches Islet, auf dem Substrat indizieren. Vorzugsweise werden zwei Teilbereiche dann als sich überlappend angesehen, wenn ihr flächiger Überlappungsgrad einen vorgegebenen Wert übersteigt. Beispielsweise kann ein Überlappungsgrad in einem Wertebereich von [0...1] ermittelt werden und dann eine Überlappung erst dann festgestellt werden, wenn der Überlappungsgrad einen Schwellenwert, z.B. den Wert 0,2, übersteigt.

Solche sich überlappenden Teilbildbereiche können prinzipiell einen gleichen bzw. ähnlichen relevanten Teilbildbereich indizieren. Daher ist es vorteilhaft, aus solchen, sich überlappenden Teilbildbereichen nur einen auszuwählen, da ein bestimmter relevanter Teilbereich eines Zellsubstrats nur einmal in die spätere Auswertung zur Bestimmung des Konfidenzmaßes eingehen soll und eben nicht mehrfach.

Figur 6 zeigt hierzu eine bevorzugte Ausführungsform des erfindungsgemäßen Verfahrens.

Die Reihenfolge der Schritte S1 und S2 ist gleich zu der Ausführungsform aus der Figur 4. In dem Schritt S2 werden die Lokalisationsinformationen LI, die ersten Teil-Konfidenzmaße ETKM und hier auch die Präsenz-Konfidenzmaße OB bestimmt, insbesondere je relevantem Teilbereich. Auf den Schritt S2 folgt dann ein Schritt S21, in welchem festgestellt wird, ob die Lokalisationsinformationen, gegeben durch das Datenelement LI, eine Menge mehrerer, sich überlappender Teilbereiche indizieren. Insbesondere wird in Schritt S21 diese Menge bestimmt. Diese Menge der sich überlappenden Teilbildbereiche und ihrer Lokalisationsinformationen können als eine Menge von Lokalisationsinformationen MLI als ein Datenelement bereitgestellt werden.

Aus der Menge der sich überlappenden Teilbildbereiche wird jener Teilbildbereich bzw. jene Lokalisationsinformation behalten, welcher bzw. welche den höchsten Wert des Präsenz-Konfidenzmaßes OB aufweist.

In einem Schritt S22 wird dann eine Lokalisationsinformation eines bestimmten Teilbildbereiches aus dieser Menge der sich überlappenden Teilbereiche ermittelt und behalten. Die Lokalisationsinformationen der anderen Teilbildbereiche aus dieser Menge der sich überlappenden Teilbildbereiche werden verworfen. Behalten werden ferner Lokalisationsinformationen, welche keine sich überlappenden Teilbildbereiche indizieren. Die behaltenen Lokalisationsinformationen sind dann die Basis zur weiteren Prozessierung. Die behaltenen Lokalisationsinformationen sind als ein Datenelement VLI dargestellt. Auf Basis der behaltenen Lokalisationsinformationen VLI kann also gewährleistet werden, dass bei sich überlappenden Teilbildbereichen, welche einen gleichen relevanten Bereich des Substrats indizieren nur ein bestimmter Teilbereich in die finale Auswertung bzw. die Prozessierung eingeht. Somit wird gewährleistet, dass ein relevanter Zellsubstratbereich nur einmal als ein Teilbildbereich in die spätere Auswertung eingeht und das Ergebnis nicht verfälscht.

In dem Schritt S3 erfolgt dann das Extrahieren der jeweiligen Teilbildbereiche aus dem Fluoreszenzbild hier auf Basis der verbleibenden bzw. behaltenen Lokalisationsinformationen VLI.

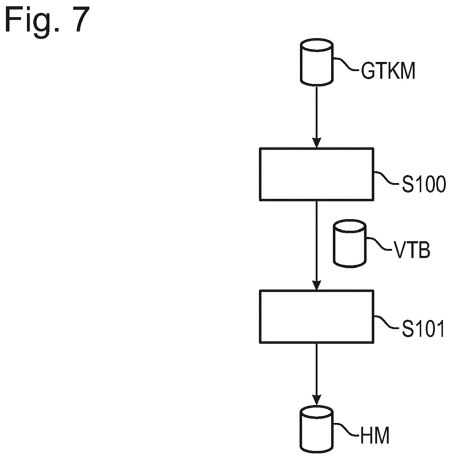

Die Figur 7 zeigt bevorzugte Schritte zur Bestimmung eines Helligkeitsmaßes der Präsenz des Fluoreszenzmusters. In einem Schritt S100 wird eine Menge von Teilbildbereichen bestimmt, welche eine Präsenz des Fluoreszenzmusters aufweisen. Beispielsweise kann auf Basis der gewichteten Teilbild-Konfidenzmaße GTKM dann eine Menge von Teilbildbereichen bestimmt werden, deren Teilbild-Konfidenzmaß GTKM bzw. deren Wert R1 einen Schwellenwert überschreitet.

Diese Menge von Teilbildbereichen bzw. verbleibenden Teilbildbereichen kann als ein Datenelement VTB dargestellt werden.

In einem Schritt S101 erfolgt dann das Ermitteln des Helligkeitsmaßes HM der Präsenz des Fluoreszenzmusters auf dem Immunfluoreszenzbild auf Basis der Menge von Teilbildbereichen VTB, welche eine Präsenz des Fluoreszenzmusters aufweisen.

Es werden also insbesondere nur Teilbildbereiche in Betracht gezogen als die Teilbildbereiche VTB, welche eine Klassenentscheidung "positive Fluoreszenz" aufweisen.

Für diese Teilbildbereiche erfolgt insbesondere eine Analyse mittels Statistik der Pixelwerte in diesen Teilbildbereichen.

Je Teilbildbereich wird ein Histogramm der Helligkeitswerte der Pixel erstellt und dann jener Helligkeitswert ermittelt, bei welchem sich ein 0,8-Quantil bzw. bei welchem 80 % der Pixel einen Intensitätswert aufweisen, welcher größer als das 0,8-Quantil ist. Dieser Wert des 0,8-Quantils ist dann zu verwenden als der Teil-Helligkeitswert eines Teilbildbereiches. Für alle zu betrachtenden Teilbildbereiche werden die jeweiligen Teilbild-Helligkeitswerte dann vorzugsweise einer Mittelung unterzogen. Es werden also vorzugsweise diese Helligkeits-Quantilwerte der jeweiligen Teilbildbereiche gemittelt. Alternativ kann eine Median-Wertbestimmung erfolgen.

Dieser mittels Median oder Mittelwert ermittelte Helligkeits-Quantilwert kann dann als das Helligkeitsmaß HM ausgegeben werden.

Besonders bevorzugt erfolgt ferner eine Quantisierung des sich ergebenden Helligkeits-Quantilwertes in vier Quantisierungsbereiche. Beispielsweise kann der Helligkeits-Quantilwert in einem Wertebereich von [0...255] liegen, sodass eine Quantisierung auf vier Quantisierungsbereiche mit Stufenwerten von [1...5] erfolgt.

Ein solcher Stufenwert kann dann an den Nutzer ausgegeben werden.

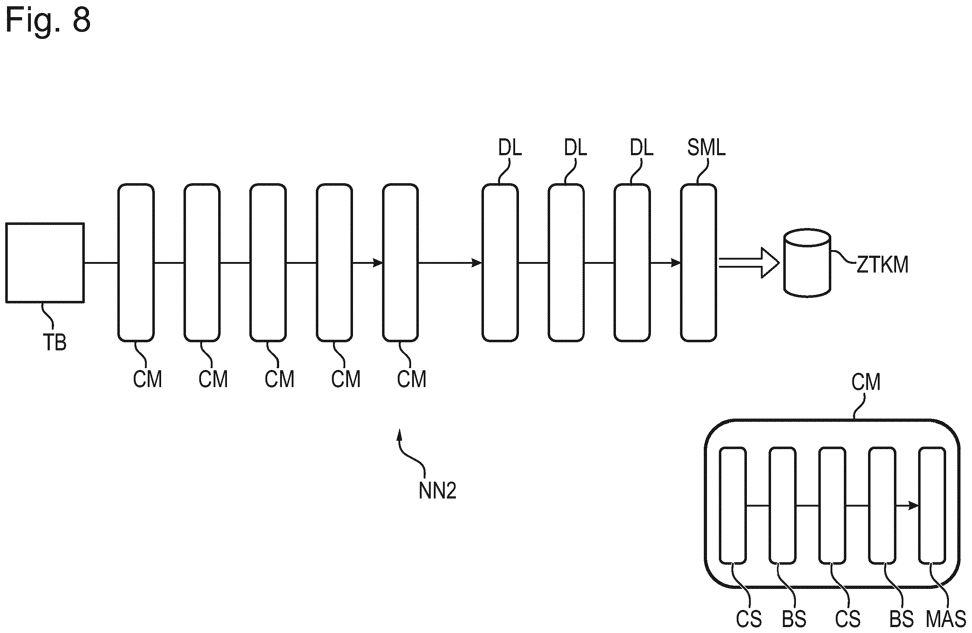

Die Figur 8 zeigt eine bevorzugte Struktur des zweiten neuronalen Netzes NN2. Das neuronale Netz NN2 analysiert ein jeweiliges Teilbild TB und bestimmt für ein jeweiliges Teilbild TB ein jeweiliges zweites Teil-Konfidenzmaß ZTKM.

Hierfür kann das neuronale Netz NN2 auf eine Abfolge jeweiliger Convolutional Module CM zurückgreifen. Ein jeweiliges Convolution Modul CM weist hierbei vorzugsweise eine Abfolge eines Convolutional Steps CS, eines Batch Normalization Steps BS, eines weiteren Convolutional Steps CS sowie eines weiteren Batch Normalization Steps BS auf, gefolgt von einem Max Pooling Step MAS.

Mehrere solcher Convolutional Module CM können aufeinander folgen.

Auf das letzte Convolutional Modul CM kann dann vorzugsweise eine weitere Prozessierung mittels mehrerer Dense Layer DL, auch Fully Connected Layer genannt. Es kann vorzugsweise mittels eines Softmax Layer SML dann das zweites Teil-Konfidenzmaß ZTKM bestimmt werden.

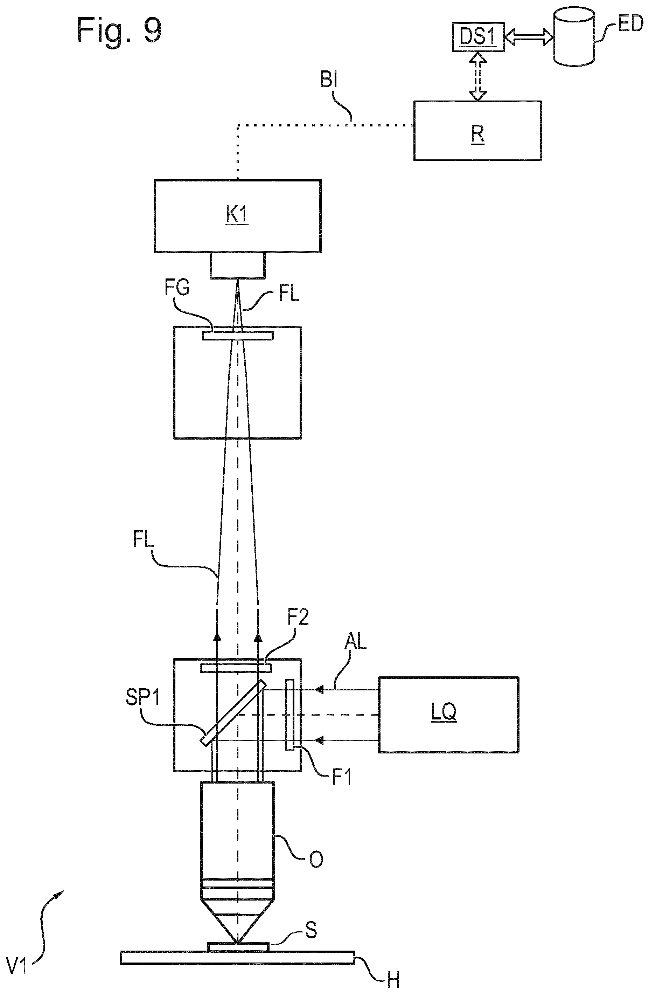

Figur 9 zeigt eine Vorrichtung V1, mittels welcher das erfindungsgemäße Verfahren vorzugsweise durchgeführt werden kann. Die Vorrichtung V1 kann als ein Fluoreszenzmikroskop bezeichnet werden. Die Vorrichtung V1 weist eine Halterung H für ein Substrat S bzw. einen Objektträger mit einem solche Substrat S auf, welches bzw. welcher in der zuvor beschriebenen Weise inkubiert wurde. Über eine Optik O wird Anregungslicht AL einer Anregungslichtquelle LQ hin zu dem Substrat S geleitet. Sich ergebende Fluoreszenzstrahlung FL wird dann durch die Optik O zurücktransmittiert und passiert den dichroitischen Spiegel SP1 und einen optionalen optischen Filter F2. Vorzugsweise passiert die Fluoreszenzstrahlung FL einen optischen Filter FG, welcher einen Grünkanal herausfiltert. Eine Kamera K1 ist vorzugsweise eine Monochromkamera, welche dann bei Vorhandensein eines optischen Filters FG die Fluoreszenzstrahlung FL in einem Grünkanal erfasst. Gemäß einer alternativen Ausführungsform ist die Kamera K1 eine Farbbildkamera, welche ohne Verwendung des optischen Filters FG auskommt und mittels einer Bayer-Matrix das Fluoreszenzbild in dem entsprechenden Farbkanal als einen Grünkanal erfasst. Die Kamera K1 stellt die Bildinformation BI bzw. das Fluoreszenzbild an eine Recheneinheit R bereit, welche diese Bildinformation BI verarbeitet. Vorzugsweise kann die Recheneinheit R über eine Datenschnittstelle DS1 Daten ED wie beispielsweise ein Fluoreszenzbild und/oder Konfidenzmaße ausgeben bzw. bereitstellen.

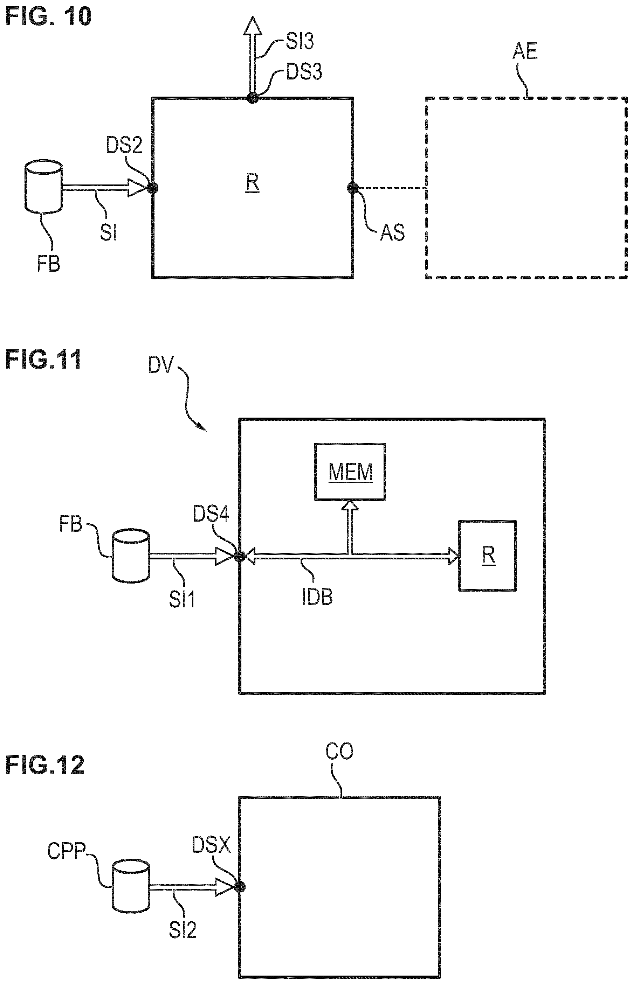

Figur 10 zeigt eine offenbarte Recheneinheit, welche vorzugsweise gemäß einer bevorzugten Ausführungsform ein Fluoreszenzbild FB als ein Datensignal SI über eine Datenschnittstelle DS2 entgennimmt. Die Recheneinheit R kann dann die zuvor beschriebenen Informationen ermitteln und über eine Datenschnittstelle DS3 als ein Datensignal SI3 bereitstellen. Vorzugsweise kann dies über ein leitungsgebundenes oder drahtloses Datennetzwerk erfolgen. Besonders bevorzugt weist die Recheneinheit R eine Ausgabeschnittstelle AS auf zur Ausgabe der Informationen über eine Ausgabeeinheit AE. Die Ausgabeeinheit AE ist vorzugsweise eine Anzeigeeinheit für eine optische Anzeige der zuvor genannten Informationen.

Figur 11 zeigt eine erfindungsgemäße Datennetzwerkvorrichtung DV gemäß einer bevorzugten Ausführungsform. Die Datennetzwerkvorrichtung DV nimmt das Fluoreszenzbild FB als ein Datensignal SI1 über eine Datenschnittstelle DS4 entgegen. Die Datennetzwerkvorrichtung DV weist eine zuvor beschriebene Recheneinheit R auf sowie eine Speichereinheit MEM. Die Recheneinheit R, eine Speichereinheit MEM als auch die Datenschnittstelle DS4 werden vorzugsweise über einen internen Datenbus IDB miteinander verbunden.

Die Figur 12 zeigt eine Ausführungsform eines vorgeschlagenen Computerprogrammprodukts CPP. Das Computerprogrammprodukt CPP kann sein Datensignal SI2 über eine Datenschnittstelle DSX durch einen Computer CO entgegengenommen werden.

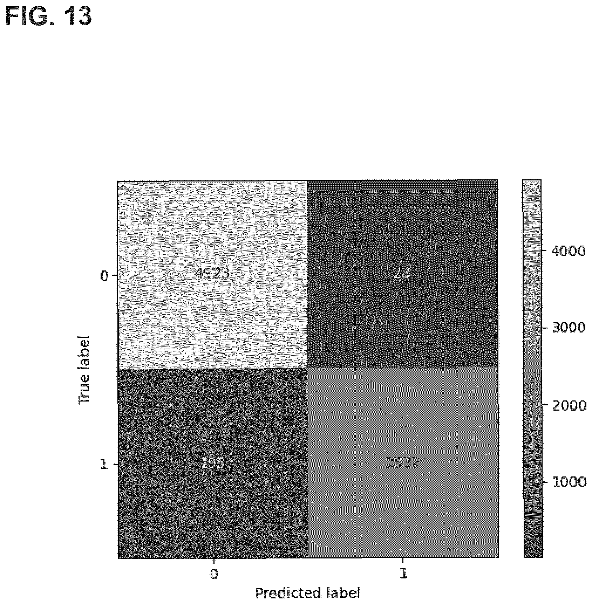

Figur 13 zeigt eine Ergebnistafel von Ergebnissen für das biologische Zellsubstrat in der Ausführungsform eines Zellabstriches von *Crithidia luciliae.*

4923 Fluoreszenzbilder, welche gemäß der Ground Truth Information keine Fluoreszenz aufweisen bzw. als "negativ" einzustufen sind, wurden auch durch das erfindungsgemäße Verfahren als negativ prädiziert.

23 negative Fluoreszenzbilder wurden durch das erfindungsgemäße Verfahren als positiv klassifiziert.

2532 Fluoreszenzbilder mit der Fluoreszenzklasse "positiv" wurden vom erfindungsgemäßen Verfahren auch als "positiv" klassifiziert.

195 Bilder der Fluoreszenzklasse "positiv" wurden als Fluoreszenzklasse "negativ" durch das erfindungsgemäße Verfahren klassifiziert.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente wie insbesondere eine Recheneinheit kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß eines Ausführungsbeispiels kann eines der Verfahren während seiner Durchführung beispielsweise dadurch umsetzen, dass dieses Speicherstellen ausliest oder in diese ein Datum oder mehrere Daten hineinschreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen.

**Patentansprüche**

1.  Verfahren zur digitalen Bildverarbeitung,
    das Verfahren aufweisend

    - Bereitstellen eines Immunfluoreszenzbildes (FB), welches eine Färbung eines biologischen Zellsubstrats (S) durch einen Fluoreszenzfarbstoff repräsentiert,
    - Bestimmen jeweiliger Lokalisationsinformationen (LI), welche jeweilige Lokalisationen jeweiliger relevanter Teilbereiche des Zellsubstrats in dem Fluoreszenzbild indizieren,
    - Extrahieren jeweiliger Teilbildbereiche (TB), welche zu den jeweiligen Teilbereichen des Zellsubstrats korrespondieren, aus dem Fluoreszenzbild auf Basis der Lokalisationsinformationen (LI),
    - Bestimmen jeweiliger zweiter Teil-Konfidenzmaße (ZTKM) jeweiliger Präsenzen des Fluoreszenzmusters auf den jeweiligen Teilbereichen mittels eines zweiten neuronalen Netzes (NN2) auf Basis der jeweiligen Teilbildbereiche (TB),
    **gekennzeichnet durch**
    - Bestimmen jeweiliger erster Teil-Konfidenzmaße (ETKM) jeweiliger Präsenzen des Fluoreszenzmusters auf den jeweiligen Teilbereichen mittels eines ersten neuronalen Netzes (NN1) auf Basis des gesamten Fluoreszenzbildes;
    - Bestimmen eines Konfidenzmaßes (KM) der Präsenz des Fluoreszenzmusters in dem Fluoreszenzbild (FB) auf Basis der ersten Teil-Konfidenzmaße (ETKM) und der zweiten Teil-Konfidenzmaße (ZTKM).

2.  Verfahren nach Anspruch 1,
    ferner aufweisend Ausgeben des Konfidenzmaßes (KM).

3. Verfahren nach Anspruch 1,
ferner aufweisend

   - Bestimmen gewichteter Teil-Konfidenzmaße (GTKM) mittels Gewichtung der ersten Teil-Konfidenzmaße (ETKM) und der zweiten Teil-Konfidenzmaße (ZTKM),
   - Bestimmen des Konfidenzmaßes auf Basis der gewichteten Teil-Konfidenzmaße.

4. Verfahren nach Anspruch 3,
ferner aufweisend Bestimmen des Konfidenzmaßes (KM) mittels Anwendung eines Schwellenwertes auf die gewichteten Teil-Konfidenzmaße (GTKM).

5. Verfahren nach Anspruch 1,
ferner aufweisend

   - Bestimmen jeweiliger Präsenz-Konfidenzmaße (OB), welche zu den jeweiligen Lokalisationen der jeweiligen relevanten Teilbereiche indizieren, zu welchem Grade an den jeweiligen Lokalisationen tatsächlich relevante Teilbereiche des Zellsubstrats präsent sind, mittels des ersten neuronalen Netzes auf Basis des gesamten Fluoreszenzbildes,
   - Feststellen, ob die Lokalisationsinformationen (LI) eine Menge (MLI) mehrerer, sich überlappender Teilbildbereiche indizieren,
   - Behalten der Lokalisationsinformation eines bestimmten Teilbildbereiches aus der Menge der sich überlappenden Teilbildbereiche auf Basis der Präsenz-Konfidenzmaße (OB) der sich überlappenden Teilbildbereiche
   und Verwerfen der Lokalisationsinformationen der anderen Teilbildbereiche aus der Menge der sich überlappenden Teilbildbereiche,
   - Extrahieren jeweiliger Teilbildbereiche aus dem Fluoreszenzbild (FB) auf Basis der verbleibenden Lokalisationsinformationen (VLI).

6. Verfahren nach Anspruch 1,
ferner aufweisend

   - Bestimmen einer Menge von Teilbildbereichen, welche eine Präsenz des Fluoreszenzmusters aufweisen,
   - Ermitteln eines Helligkeitswertes (HM) der Präsenz der Fluoreszenzmusters auf dem Immunfluoreszenzbild auf Basis der Menge von Teilbildbereichen, welche eine Präsenz des Fluoreszenzmusters aufweisen.

7. Computerprogrammprodukt (CPP), umfassend Befehle, die bei der Ausführung des Programms durch einen Computer diesen veranlassen, das Verfahren zur digitalen Bildverarbeitung nach Anspruch 1 durchzuführen.

8. Datenträgersignal (SI2), welches das Computerprogrammprodukt (CPP) nach Anspruch 7 überträgt.

9. Vorrichtung zur Detektion wenigstens eines Fluoreszenzmusters auf einem Immunfluoreszenzbild (FB) eines biologischen Zellsubstrats,

   aufweisend

   - eine Haltevorrichtung (H) für einen Objektträger mit dem Zellsubstrat(S), welches mit einer Patientenprobe, aufweisend Autoantikörper, sowie ferner mit sekundären Antikörpern, welche jeweils mit einem Fluoreszenzfarbstoff markiert sind, inkubiert wurde,
   - wenigstens eine Bilderfassungseinheit (K1) zum Erfassen eines Fluoreszenzbildes (SG) des Zellsubstrats (S)

   sowie ferner aufweisend wenigstens eine Recheneinheit (R), welche ausgebildet ist, die Schritte auszuführen

   - Bestimmen jeweiliger Lokalisationsinformationen (LI), welche jeweilige Lokalisationen jeweiliger relevanter Teilbereiche des Zellsubstrats in dem Fluoreszenzbild (FB) indizieren,
   - Extrahieren jeweiliger Teilbildbereiche, welche zu den jeweiligen Teilbereichen des Zellsubstrats korrespondieren, auf Basis der Lokalisationsinformationen (LI),
   - Bestimmen jeweiliger zweiter Teil-Konfidenzmaße (ZTKM) jeweiliger Präsenzen des Fluoreszenzmusters auf den jeweiligen Teilbereichen mittels eines zweiten neuronalen Netzes (NN2) auf Basis der jeweiligen Teilbildbereiche,
   **gekennzeichnet durch** die Schritte:
   - Bestimmen jeweiliger erster Teil-Konfidenzmaße (ETKM) jeweiliger Präsenzen des Fluoreszenzmusters auf den jeweiligen Teilbereichen mittels eines ersten neuronalen Netzes (NN1) auf Basis des gesamten Fluoreszenzbildes; und
   - Bestimmen eines Konfidenzmaßes (KM) der Präsenz des Fluoreszenzmusters in dem Fluoreszenzbild (FB) auf Basis der ersten Teil-Konfidenzmaße (ETKM) und der zweiten Teil-Konfidenzmaße (ZTKM).

10. Datennetzwerkvorrichtung (DV),

   aufweisend wenigstens eine Datenschnittstelle (DS4) zum Entgegennehmen eines Fluoreszenzbildes (FB), welches eine Färbung eines Zellsubstrats durch einen Fluoreszenzfarbstoff repräsentiert,

sowie ferner aufweisend wenigstens eine Recheneinheit (R), welche ausgebildet ist im Zuge einer digitalen Bildverarbeitung die Schritte auszuführen

- Bestimmen jeweiliger Lokalisationsinformationen (LI), welche jeweilige Lokalisationen jeweiliger relevanter Teilbereiche des Zellsubstrats (S) in dem Fluoreszenzbild (FB) indizieren,
- Extrahieren jeweiliger Teilbildbereiche (TB), welche zu den jeweiligen Teilbereichen des Zellsubstrats korrespondieren, auf Basis der Lokalisationsinformationen (LI),
- Bestimmen jeweiliger zweiter Teil-Konfidenzmaße (ZTKM) jeweiliger Präsenzen des Fluoreszenzmusters auf den jeweiligen Teilbereichen mittels eines zweiten neuronalen Netzes (NN2) auf Basis der jeweiligen Teilbildbereiche,
**gekennzeichnet durch** die Schritte:
- Bestimmen jeweiliger erster Teil-Konfidenzmaße (ETKM) jeweiliger Präsenzen des Fluoreszenzmusters auf den jeweiligen Teilbereichen mittels eines ersten neuronalen Netzes (NN1) auf Basis des gesamten Fluoreszenzbildes; und
- Bestimmen eines Konfidenzmaßes (KM) der Präsenz des Fluoreszenzmusters in dem Fluoreszenzbild auf Basis der ersten Teil-Konfidenzmaße (ETKM) und der zweiten Teil-Konfidenzmaße.

**Claims**

1. Method for digital image processing,
   the method comprising

   - providing an immunofluorescence image (FB), which represents a staining of a biological cell substrate (S) by a fluorescence stain,
   - determining respective items of location information (LI), which indicate respective locations of respective relevant subsections of the cell substrate in the fluorescence image,
   - extracting respective image subsections (TB), which correspond to the respective subsections of the cell substrate, from the fluorescence image on the basis of the items of location information (LI),
   - determining respective second partial confidence measures (ZTKM) of respective presences of the fluorescence pattern on the respective subsections using a second neural network (NN2) on the basis of the respective image subsections (TB),
   **characterized in that**
   - determining respective first partial confidence measures (ETKM) of respective presences of the fluorescence pattern on the respective subsections using a first neural network (NN1) on the basis of the overall fluorescence image
   - determining a confidence measure (KM) of the presence of the fluorescence pattern in the fluorescence image (FB) on the basis of the first partial confidence measures (ETKM) and the second partial confidence measures (ZTKM).

2. Method according to Claim 1,
   furthermore comprising outputting the confidence measure (KM).

3. Method according to Claim 1,
   furthermore comprising

   - determining weighted partial confidence measures (GTKM) via weighting of the first partial confidence measures (ETKM) and the second partial confidence measures (ZTKM),
   - determining the confidence measure on the basis of the weighted partial confidence measures.

4. Method according to Claim 3,
   furthermore comprising determining the confidence measure (KM) via application of a threshold value to the weighted partial confidence measures (GTKM).

5. Method according to Claim 1,
   furthermore comprising

   - determining respective presence confidence measures (OB), which indicate for the respective locations of the respective relevant subsections to which degrees at the respective locations actually relevant subsections of the cell substrate are present, using the first neural network on the basis of the overall fluorescence image,
   - determining whether the items of location information (LI) indicate a set (MLI) of multiple overlapping image subsections,
   - retaining the item of location information of a specific image subsection from the set of overlapping image subsections on the basis of the presence confidence measures (OB) of the overlapping image subsections
   and discarding the items of location information of the other image subsections from the set of overlapping image subsections,

- extracting respective image subsections from the fluorescence image (FB) on the basis of the remaining items of location information (VLI).

6. Method according to Claim 1, furthermore comprising

- determining a set of image subsections which comprise a presence of the fluorescence pattern,
- ascertaining a brightness value (HM) of the presence of the fluorescence pattern on the immunofluorescence image on the basis of the set of image subsections which comprise a presence of the fluorescence pattern.

7. Computer program product (CPP), comprising commands which, upon the execution of the program by a computer, prompt it to carry out the method for digital image processing according to Claim 1.

8. Data carrier signal (SI2), which transmits the computer program product (CPP) according to Claim 7.

9. Device for detecting at least one fluorescence pattern on an immunofluorescence image (FB) of a biological cell substrate, comprising

- a holding device (H) for an object carrier having the cell substrate (S), which was incubated with a patient sample, including autoantibodies, and furthermore with secondary antibodies, which are each marked using a fluorescence stain,
- at least one image capture unit (K1) for capturing a fluorescence image (SG) of the cell substrate (S)

and furthermore comprising at least one computing unit (R), which is designed to execute the following steps

- determining respective items of location information (LI), which indicate respective locations of respective relevant subsections of the cell substrate in the fluorescence image (FB),
- extracting respective image subsections, which correspond to the respective subsections of the cell substrate, on the basis of the items of location information (LI),
- determining respective second partial confidence measures (ZTKM) of respective presences of the fluorescence pattern on the respective subsections using a second neural network (NN2) on the basis of the respective image subsections,
**characterized by** the steps:

- determining respective first partial confidence measures (ETKM) of respective presences of the fluorescence pattern on the respective subsections using a first neural network (NN1) on the basis of the overall fluorescence image, and
- determining a confidence measure (KM) of the presence of the fluorescence pattern in the fluorescence image (FB) on the basis of the first partial confidence measures (ETKM) and the second partial confidence measures (ZTKM).

10. Data network device (DV),

comprising at least one data interface (DS4) for accepting a fluorescence image (FB), which represents a staining of a cell substrate by a fluorescence stain, and furthermore comprising at least one computing unit (R), which is designed to execute the following steps in the course of digital image processing

- determining respective items of location information (LI), which indicate respective locations of respective relevant subsections of the cell substrate (S) in the fluorescence image (FB),
- extracting respective image subsections (TB), which correspond to the respective subsections of the cell substrate, on the basis of the items of location information (LI),
- determining respective second partial confidence measures (ZTKM) of respective presences of the fluorescence pattern on the respective subsections using a second neural network (NN2) on the basis of the respective image subsections,
**characterized by** the steps:

- determining respective first partial confidence measures (ETKM) of respective presences of the fluorescence pattern on the respective subsections using a first neural network (NN1) on the basis of the overall fluorescence image (FB), and
- determining a confidence measure (KM) of the presence of the fluorescence pattern in the fluorescence image on the basis of the first partial confidence measures (ETKM) and the second partial confidence measures.

**Revendications**

1. Procédé de traitement d'image numérique,

le procédé comprenant

- la fourniture d'une image immunofluorescente (FB) qui représente une coloration d'un substrat de cellule biologique (S) par un colorant fluorescent,
- la détermination d'informations de localisation respectives (LI) qui indiquent des localisations respectives de zones partielles pertinentes respectives du substrat de cellule dans l'image fluorescente,
- l'extraction de zones d'image partielles respectives (TB) qui correspondent aux zones partielles respectives du substrat de cellule à partir de l'image fluorescente sur la base des informations de localisation (LI),
- la détermination de secondes mesures de confiance partielles respectives (ZTKM) de présences respectives du motif fluorescent sur les zones partielles respectives au moyen d'un second réseau neuronal (NN2) sur la base des zones d'image partielles respectives (TB),

**caractérisé par**

- la détermination de premières mesures de confiance partielles respectives (ETKM) de présences respectives du motif fluorescent sur les zones partielles respectives au moyen d'un premier réseau neuronal (NN1) sur la base de l'image fluorescente entière,
- la détermination d'une mesure de confiance (KM) de la présence du motif fluorescent dans l'image fluorescente (FB) sur la base des premières mesures de confiance partielles (ETKM) et des secondes mesures de confiance partielles (ZTKM).

2. Procédé selon la revendication 1, comprenant en outre la fourniture en sortie de la mesure de confiance (KM).

3. Procédé selon la revendication 1, comprenant en outre

- la détermination de mesures de confiance partielles pondérées (GTKM) au moyen d'une pondération des premières mesures de confiance partielles (ETKM) et des secondes mesures de confiance partielles (ZTKM),
- la détermination de la mesure de confiance sur la base des mesures de confiance partielles pondérées.

4. Procédé selon la revendication 3, comprenant en outre la détermination de la mesure de confiance (KM) au moyen de l'application d'un seuil aux mesures de confiance partielles pondérées (GTKM).

5. Procédé selon la revendication 1, comprenant en outre

- la détermination de mesures de confiance de présence respectives (OB) qui indiquent pour les localisations respectives des zones partielles pertinentes respectives, à quel degré des zones partielles du substrat de cellule réellement pertinentes sont présentes aux localisations respectives, au moyen du premier réseau neuronal et sur la base de l'image fluorescente entière,
- la détermination du fait de savoir si les informations de localisation (LI) indiquent un ensemble (MLI) de plusieurs zones d'image partielles se chevauchant,
- la conservation des informations de localisation d'une zone d'image partielle déterminée de l'ensemble des zones d'image partielles se chevauchant sur la base des mesures de confiance de présence (OB) des zones d'image partielles se chevauchant,
et le rejet des informations de localisation des autres zones d'image partielles de l'ensemble des zones d'image partielles se chevauchant,
- l'extraction de zones d'image partielles respectives de l'image fluorescente (FB) sur la base des informations de localisation restantes (VLI).

6. Procédé selon la revendication 1, comprenant en outre

- la détermination d'un ensemble de zones d'image partielles qui présentent une présence du motif fluorescent,
- la détermination d'une valeur de luminosité (HM) de la présence du motif fluorescent sur l'image immunofluorescente sur la base de l'ensemble de zones d'image partielles qui présentent une présence du motif fluorescent.

7. Produit de programme informatique (CPP), comprenant des instructions qui, lors de l'exécution du programme par un ordinateur, l'amènent à mettre en œuvre le procédé de traitement d'image numérique selon la revendication 1.

8. Signal porteur de données (SI2), qui transmet le produit de programme informatique (CPP) selon la revendication 7.

9. Dispositif de détection d'au moins un motif fluorescent sur une image immunofluorescente (FB) d'un substrat de cellule biologique,
comprenant

   - un dispositif de maintien (H) pour une lame porte-objet dotée du substrat de cellule (S), qui a été amené à incuber avec un échantillon de patient comprenant des auto-anticorps, et comprenant en outre des anticorps secondaires, qui sont respectivement marqués par un colorant fluorescent,
   - au moins une unité d'acquisition d'image (K1) pour l'acquisition d'une image fluorescente (SG) du substrat de cellule (S)
   et comprenant en outre au moins une unité de calcul (R), qui est conçue pour exécuter les étapes comprenant
   - la détermination d'informations de localisation respectives (LI) qui indiquent des localisations respectives de zones partielles pertinentes respectives du substrat de cellule dans l'image fluorescente (FB),
   - l'extraction de zones d'image partielles respectives qui correspondent aux zones partielles respectives du substrat de cellule sur la base des informations de localisation (LI),
   - la détermination de secondes mesures de confiance partielles respectives (ZTKM) de présences respectives du motif fluorescent sur les zones partielles respectives au moyen d'un second réseau neuronal (NN2) sur la base des zones d'image partielles respectives,

   **caractérisé par** les étapes comprenant :

   - la détermination de premières mesures de confiance partielles respectives (ETKM) de présences respectives du motif fluorescent sur les zones partielles respectives au moyen d'un premier réseau neuronal (NN1) sur la base de l'image fluorescente entière ; et
   - la détermination d'une mesure de confiance (KM) de la présence du motif fluorescent dans l'image fluorescente (FB) sur la base des premières mesures de confiance partielles (ETKM) et des secondes mesures de confiance partielles (ZTKM).

10. Dispositif de réseau de données (DV),

   comprenant au moins une interface de données (DS4) pour la réception d'une image fluorescente (FB), qui représente une coloration d'un substrat de cellule par un colorant fluorescent,

et comprenant en outre au moins une unité de calcul (R), qui est conçue pour exécuter, dans le cadre d'un traitement d'image numérique, les étapes comprenant

   - la détermination d'informations de localisation respectives (LI) qui indiquent des localisations respectives de zones partielles pertinentes respectives du substrat de cellule (S) dans l'image fluorescente (FB),
   - l'extraction de zones d'image partielles respectives (TB) qui correspondent aux zones partielles respectives du substrat de cellule sur la base des informations de localisation (LI),
   - la détermination de secondes mesures de confiance partielles respectives (ZTKM) de présences respectives du motif fluorescent sur les zones partielles respectives au moyen d'un second réseau neuronal (NN2) sur la base des zones d'image partielles respectives,

   **caractérisé par** les étapes comprenant :

   - la détermination de premières mesures de confiance partielles respectives (ETKM) de présences respectives du motif fluorescent sur les zones partielles respectives au moyen d'un premier réseau neuronal (NN1) sur la base de l'image fluorescente entière ; et
   - la détermination d'une mesure de confiance (KM) de la présence du motif fluorescent dans l'image fluorescente sur la base des premières mesures de confiance partielles (ETKM) et des secondes mesures de confiance partielles.

Fig.1
a) FB1
b) FB2

Fig.2

a) TBA FB1

b) FB2 TBB1 TBB2 TBB3

c) TBB3 TBB2 TBB1

Fig.3

a)

FB11

b)

FB12

c)

TBB13

TBB12

TBB11

EP 4 345 658 B1

Fig. 4

**Fig. 5**

**Fig. 6**

Fig. 7

Fig. 8

TB CM CM CM CM CM DL DL DL SML ZTKM

NN2

CM

CS BS CS BS MAS

EP 4 345 658 B1

# Fig. 9

FIG. 10

FIG.11

FIG.12

# FIG. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3971827 A1
- EP 4030340 A1
- CN 108364006 A

**In der Beschreibung aufgeführte Nicht-Patentliteratur**

- **J. REDMON** ; **S. DIVVALA** ; **R. GIRSHICK** ; **A. FARHADI**. You Only Look Once: Unified, Real-Time Object Detection. *IEEE Conference on Computer Vision and Pattern Recognition (CVPR)*, 2016, vol. 2016, 779-788